# EUROPEAN PATENT APPLICATION

(11) **EP 4 699 528 A1**
(43) Date of publication of application: **25.02.2026**
(21) Application number: 25196727.9
(22) Date of filing: 19.08.2025
(51) Int. Cl.: A61B 5/055, G01R 33/28, A61B 5/00

(54) **MEDICAL IMAGING APPARATUS, MEDICAL IMAGING SUPPORT METHOD, INFORMATION PROCESSING APPARATUS, AND PROGRAM**

(30) Priority: 19.08.2024 JP 2024137861
(71) Applicant: FUJIFILM Corporation, Tokyo 106-8620 (JP)
(72) Inventor: KITAMOTO, Shizue, Tokyo, 1068620 (JP); HAMADA, Kota, Tokyo, 1068620 (JP); UCHIO, Yoshiki, Tokyo, 1068620 (JP)
(74) Representative: Dehns Germany Partnerschaft mbB

(57) **Abstract**

There is provided a medical imaging apparatus that captures a medical image of a subject, including: a sensor that acquires information used to identify a body position of the subject; and a processor, in which the processor is configured to: display a registration screen for accepting registration of the body position of the subject on a display device; accept an imaging start instruction for the subject whose body position has been registered; identify the body position of the subject based on the information obtained from the sensor; and in a case where the registration screen has transitioned to an examination window screen including an image display area for displaying the medical image, and the identified body position and the registered body position are different from each other, display a pop-up screen including a proposal for re-registration of the body position on the examination window screen.

## Description

### BACKGROUND OF THE INVENTION

### 1. Field of the Invention

The present disclosure relates to a medical imaging apparatus, a medical imaging support method, an information processing apparatus, and a program.

### 2. Description of the Related Art

JP2014-121364A discloses a radiographic tomography apparatus comprising means for recognizing a body position of a subject by using a depth sensor, determining appropriateness of scan conditions such as a body position set in advance, and issuing a notification that the conditions are inappropriate in a case where such a determination is made.

JP2021-121092A discloses a method of acquiring a depth image of a patient on a table of a medical imaging system via a depth camera, correcting the depth image based on histogram data from the depth image, and extracting a three-dimensional structure of the patient from the corrected depth image. In addition, JP2021-121092A describes a configuration in which a patient's posture is determined from the extracted three-dimensional structure, and a warning is output in a case where the posture does not match a desired posture of the patient for performing a scan protocol to be used, based on a comparison. The term "patient" is synonymous with "subject", and the term "patient's posture" is synonymous with "subject's body position".

### SUMMARY OF THE INVENTION

In an examination using a medical imaging apparatus typified by a magnetic resonance imaging (MRI) apparatus, a user such as a technician launches a subject registration screen on a display device of a console and pre-registers subject information, an examination site, a body position of the subject, an imaging protocol, and the like on the subject registration screen.

After that, the user sets the subject on a patient table in a shielded room and starts the examination by pressing a start button for issuing an instruction to start imaging. Before or after the start button is pressed, the screen of the console transitions to an examination window screen. That is, in a case where the instruction to start imaging is issued, the screen of the console has already transitioned to the examination window screen, and in principle, imaging should be executed, and the image reconstructed from data obtained by the imaging should be output to the examination window screen.

However, for some reason, imaging may proceed with the subject in a body position different from the pre-registered body position (hereinafter, referred to as a "registered body position"). For example, suddenly due to patient requests or the like, imaging may be performed in a body position different from the registered body position. Additionally, it is also assumed that the user may register an incorrect body position in a case where the body position of the subject is pre-registered. Alternatively, it is also assumed that the registered body position is correct, but the subject is set on the patient table in a body position different from the registered body position in a case where the subject is set on the patient table. In a case where imaging proceeds with a body position different from the registered body position, it may result in the need for re-imaging.

In this regard, according to the technologies disclosed in JP2014-121364A and JP2021-121092A, a configuration is employed in which a notification is issued in a case where it is found that the body position is different from the registered body position. Therefore, the user is expected to notice the error, re-register the correct body position, and perform appropriate imaging.

On the other hand, in the related art, in order to change the registered body position to the correct body position, it is necessary to return from the examination window screen to the subject registration screen and re-register the body position, making a re-registration operation cumbersome. In addition, JP2014-121364A and JP2021-121092A only provide means for issuing a notification of a mismatch in body positions and do not disclose specific means for resolving the mismatch.

The present disclosure has been made in view of such circumstances, and an object of the present disclosure is to provide a medical imaging apparatus, a medical imaging support method, an information processing apparatus, and a program that can enhance user convenience and capture a medical image of a subject in an appropriate body position.

According to a first aspect of the present disclosure, there is provided a medical imaging apparatus that captures a medical image of a subject, including: a sensor that acquires information used to identify a body position of the subject; and a processor, in which the processor is configured to: display a registration screen for accepting registration of the body position of the subject on a display device; accept an imaging start instruction for the subject whose body position has been registered; identify the body position of the subject based on the information obtained from the sensor; and in a case where the registration screen has transitioned to an examination window screen including an image display area in which the medical image is displayed, and the identified body position and the registered body position are different from each other, display a pop-up screen including a proposal for re-registration of the body position on the examination window screen.

According to the first aspect, in a case where a body position different from the registered body position is identified in a state in which the examination window screen is displayed on the display device, the pop-up screen is displayed on the examination window screen. This pop-up screen includes a proposal for the body position to be recommended for re-registration in place of the registered body position, and the user can determine whether to accept or reject the presented proposal and re-register an appropriate body position with a simple operation.

According to a second aspect, in the medical imaging apparatus according to the first aspect, a configuration may be employed in which the sensor includes a camera, and the information used to identify the body position of the subject includes an image captured by the camera.

According to a third aspect, in the medical imaging apparatus according to the first or second aspect, a configuration may be employed in which a patient table including a top plate that moves with the subject placed thereon; and a gantry including an imaging space into which the subject is inserted by movement of the top plate are further provided.

According to a fourth aspect, in the medical imaging apparatus according to the third aspect, a configuration may be employed in which the patient table includes a plurality of connectors to which a receive coil that receives a signal emitted by the subject is connected, and the sensor detects at least one of a position of the connector to which the receive coil is connected or a type of the receive coil.

According to a fifth aspect, in the medical imaging apparatus according to any one of the first to fourth aspects, a configuration may be employed in which the pop-up screen includes a part of the registration screen.

According to a sixth aspect, in the medical imaging apparatus according to any one of the first to fifth aspects, the processor may be configured to accept re-registration of the body position of the subject on the pop-up screen.

According to a seventh aspect, in the medical imaging apparatus according to any one of the first to sixth aspects, the registration screen may be a screen for accepting registration of imaging conditions including the body position of the subject and registration of subject information.

According to an eighth aspect, in the medical imaging apparatus according to any one of the first to fifth aspects, a configuration may be employed in which the registered body position and a proposed body position for re-registration are displayed on the pop-up screen.

According to a ninth aspect, in the medical imaging apparatus according to any one of the first to eighth aspects, the processor may be configured to display a proposed body position corresponding to the identified body position on the pop-up screen.

According to a tenth aspect, in the medical imaging apparatus according to any one of the first to ninth aspects, a configuration may be employed in which a button for issuing an instruction to continue imaging is displayed on the pop-up screen.

According to an eleventh aspect, in the medical imaging apparatus according to any one of the first to tenth aspects, the processor may be configured to display the pop-up screen within the image display area on the examination window screen.

According to a twelfth aspect, in the medical imaging apparatus according to any one of the first to eleventh aspects, the processor may be configured to: in a case where the examination window screen is being displayed, display information indicating that identification of the body position of the subject is being performed on the examination window screen during a period until the identification of the body position is completed.

According to a thirteenth aspect, in the medical imaging apparatus according to any one of the first to twelfth aspects, the processor may be configured to: in a case where the body position of the subject is not identified, display a screen including the information obtained from the sensor on the examination window screen as a pop-up.

According to a fourteenth aspect, in the medical imaging apparatus according to the thirteenth aspect, a configuration may be employed in which the information obtained from the sensor includes an image captured by a camera as the sensor.

According to a fifteenth aspect, in the medical imaging apparatus according to the thirteenth or fourteenth aspect, a configuration may be employed in which the information obtained from the sensor includes coil information including at least one of information indicating a type of a receive coil that receives a signal emitted by the subject or information indicating a position of a connector to which the receive coil is connected.

According to a sixteenth aspect, in the medical imaging apparatus according to any one of the thirteenth to fifteenth aspects, a configuration may be employed in which the screen including the information obtained from the sensor displays the registered body position, and a body position as a candidate for re-registration.

According to a seventeenth aspect, in the medical imaging apparatus according to any one of the thirteenth to sixteenth aspects, a configuration may be employed in which the screen including the information obtained from the sensor includes a part of the registration screen.

According to an eighteenth aspect, there is provided a medical imaging support method executed by a processor, comprising: causing the processor to execute: displaying a registration screen for accepting registration of a body position of a subject on a display device; accepting an imaging start instruction for capturing a medical image of the subject whose body position has been registered; identifying the body position of the subject based on information obtained from a sensor; and in a case where the registration screen has transitioned to an examination window screen including an image display area for displaying the medical image of the subject, and the identified body position and the registered body position are different from each other, displaying a pop-up screen including a proposal for re-registration of the body position on the examination window screen.

The processor may automatically execute processing of each operation in accordance with a program, or may accept an input of an instruction from a user for a part or all of the processing and execute the processing according to the accepted instruction. In addition, the order of processing can be changed as long as no technical inconsistencies arise.

For the medical imaging support method according to the eighteenth aspect, a configuration can be employed in which the same specific aspect as the medical imaging apparatus according to any one of the second to eighteenth aspects is included.

According to a nineteenth aspect, there is provided an information processing apparatus comprising: a processor, in which the processor is configured to: display a registration screen for accepting registration of a body position of a subject on a display device; accept an imaging start instruction for capturing a medical image of the subject whose body position has been registered; identify the body position of the subject based on information obtained from a sensor; and in a case where the registration screen has transitioned to an examination window screen including an image display area for displaying the medical image of the subject, and the identified body position and the registered body position are different from each other, display a pop-up screen including a proposal for re-registration of the body position on the examination window screen.

For the information processing apparatus according to the nineteenth aspect, a configuration can be employed in which the same specific aspect as the medical imaging apparatus according to any one of the second to eighteenth aspects is included.

A program according to a twentieth aspect is a program for causing a computer to function as the information processing apparatus according to the nineteenth aspect. The present disclosure also includes a non-transitory and tangible computer-readable storage medium storing the program according to the twentieth aspect. In the program according to the twentieth aspect and the storage medium storing the program according to the twentieth aspect, a configuration can be employed in which the same specific aspect as the medical imaging apparatus according to any one of the second to eighteenth aspects is included.

According to the present disclosure, it is possible to enhance user convenience and to capture a medical image of a subject in an appropriate body position.

### BRIEF DESCRIPTION OF THE DRAWINGS

Fig. 1 is a perspective view showing an external appearance of an exemplary MRI apparatus.
Fig. 2 is a diagram showing a schematic configuration of an inside of the MRI apparatus according to an embodiment.
Fig. 3 is a diagram showing another example of a disposition position of a camera in the MRI apparatus.
Fig. 4 is a block diagram showing an example of a hardware configuration of an information processing apparatus used in the MRI apparatus.
Fig. 5 is an example of a subject registration screen.
Fig. 6 is an example of an examination window screen.
Fig. 7 is a flowchart showing an example of an operation by the MRI apparatus.
Fig. 8 is an example of a pop-up screen that is popped up on the examination window screen.
Fig. 9 is a screen example showing an example of a display position of the pop-up screen on the examination window screen.
Fig. 10 is a display example of a screen including a camera image and coil information.
Fig. 11 is a schematic plan view of a patient table on which a subject wearing a receive coil, which is a Neuro Coil for a head and neck, is placed.
Fig. 12 is a block diagram showing a functional configuration of the information processing apparatus that performs a medical imaging support method according to the embodiment.

### DESCRIPTION OF THE PREFERRED EMBODIMENTS

Preferred embodiments of the present invention will be described below in detail with reference to the accompanying drawings. In the present specification, identical reference numerals are denoted by identical components, and duplicate descriptions will be omitted as appropriate.

### Overview of Magnetic Resonance Imaging (MRI) Apparatus

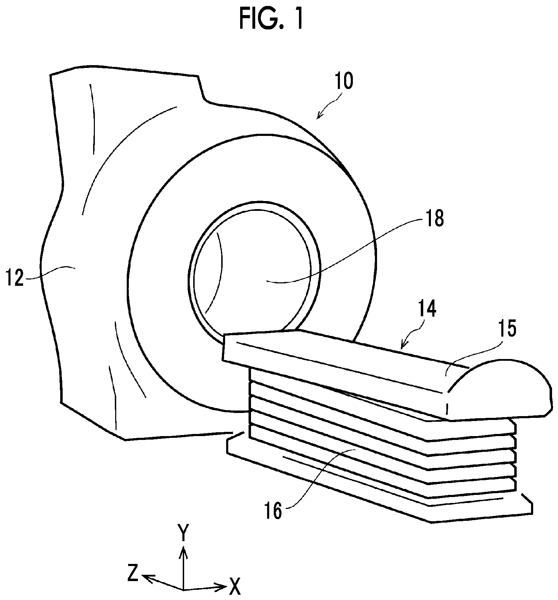

Fig. 1 is a perspective view showing an external appearance of an exemplary MRI apparatus 10. The MRI apparatus 10 comprises a gantry 12, which is an imaging apparatus main body, and a patient table 14. The gantry 12 includes a cylindrical imaging space 18, which is called a bore. A static magnetic field generating magnet, a gradient magnetic field coil, and various other coils for generating a magnetic field in the imaging space 18 are disposed in the gantry 12.

The patient table 14 comprises a top plate 15 and a leg portion 16 that supports the top plate 15, and is disposed on a front side of the gantry 12. The top plate 15 can be advanced into the imaging space 18 and retracted from the imaging space 18 by a drive mechanism (not shown) provided in the leg portion 16. The patient table 14 may be fixed to the gantry 12 or may be a movable dockable patient table that is attachable to and detachable from the gantry 12.

In the MRI apparatus 10, three axes of the apparatus, which are three-dimensional orthogonal coordinate axes in a real space including the imaging space 18, are defined. A direction of each axis of the three axes of the apparatus is uniquely determined from the gantry 12 and the patient table 14. In Fig. 1, a Z direction is a static magnetic field direction, a Y direction is a vertical direction, and an X direction is a direction orthogonal to the Y direction and the Z direction.

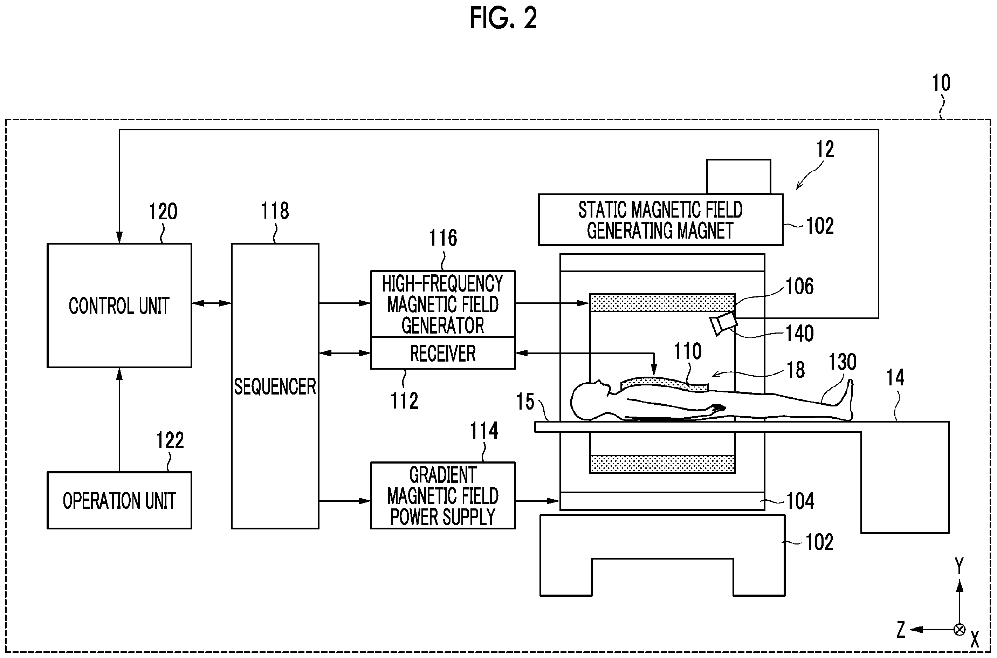

Fig. 2 is a diagram showing a schematic configuration of an inside of the MRI apparatus 10. The MRI apparatus 10 comprises a static magnetic field generating magnet 102, a gradient magnetic field coil 104, a radio frequency (RF) transmit coil 106, a receive coil 110, a receiver 112, a gradient magnetic field power supply 114, a high-frequency magnetic field generator 116, a sequencer 118, a control unit 120, and an operation unit 122. In addition, the MRI apparatus 10 comprises a camera 140 for identifying a body position of a subject 130.

The static magnetic field generating magnet 102 generates a uniform static magnetic field in the imaging space 18. The static magnetic field generating magnet 102 includes a static magnetic field generating source of a permanent magnet type, a normal conducting type, or a superconducting type. The gradient magnetic field coil 104 generates a gradient magnetic field in the imaging space 18. The gradient magnetic field coil 104 is composed of gradient magnetic field coils in three-axis directions of X, Y, and Z, which correspond to a real space coordinate system (stationary coordinate system). Each of the gradient magnetic field coils is connected to a gradient magnetic field power supply 114 and is supplied with a current. As a result, gradient magnetic fields are generated in the three-axis directions of X, Y, and Z.

The subject 130 is placed on the top plate 15 of the patient table 14, and the receive coil 110 corresponding to an examination site is worn by the subject 130. By moving the top plate 15 with the subject 130 placed thereon into the imaging space 18, the examination site of the subject 130 is positioned at a static magnetic field center in the imaging space 18.

The camera 140 is an example of a sensor from which information regarding the body position of the subject 130 is obtained. The camera 140 is typically an optical camera including an imaging optical system that includes a lens, and an imaging element that captures an optical image formed by the imaging optical system and that converts the optical image into an electrical signal. The imaging element is configured, for example, by using a complementary metal-oxide semiconductor (CMOS) type color image sensor. The imaging element is not limited to the CMOS type and may be a charge-coupled device (CCD) type image sensor. Additionally, the camera 140 may include an image processing circuit that processes the electrical signal obtained from the imaging element to form a digital image. The subject 130 on the patient table 14 is imaged by the camera 140, and the body position of the subject 130 is identified based on the image acquired via the camera 140. In the present specification, the image acquired via the camera 140 is referred to as a "camera image". An infrared camera, a millimeter-wave camera, or the like may be used instead of or in combination with the camera 140.

The RF transmit coil 106 is a coil that irradiates the subject 130 with an RF pulse which is a high-frequency magnetic field pulse. The RF transmit coil 106 is connected to the high-frequency magnetic field generator 116 and is supplied with a high-frequency pulse current from the high-frequency magnetic field generator 116.

The sequencer 118 sends commands to the high-frequency magnetic field generator 116 and the gradient magnetic field power supply 114, in accordance with an imaging pulse sequence. As a result, signals, each amplified appropriately, are sent to the RF transmit coil 106 and the gradient magnetic field coil 104.

The high-frequency magnetic field generator 116 is driven in accordance with the command from the sequencer 118, modulates the amplitude of a high-frequency pulse, and supplies the amplified high-frequency pulse current to the RF transmit coil 106. The RF transmit coil 106 applies a pulsed high-frequency magnetic field (RF pulse) to the subject 130 in accordance with the signal from the high-frequency magnetic field generator 116. Preferably, the sequencer 118 sends the command to the receive coil 110 in accordance with the imaging pulse sequence and turns off the receive coil 110 during a period when the RF pulse is applied.

By the application of the high-frequency magnetic field, nuclear magnetic resonance (NMR) phenomena are induced in spins of atoms constituting biological tissues of the subject 130. The receive coil 110 is a multi-channel RF coil unit that includes a plurality of coil elements for receiving nuclear magnetic resonance signals (NMR signals) generated from the subject 130. In Fig. 2, an example of a blanket-type receive coil 110 that is applied to imaging of a chest and an abdomen is shown, but a form of the receive coil 110 may be applied differently depending on the examination site. For example, receive coils for imaging various sites, such as a head, a spine, an abdomen, a leg, and an arm, can be used.

The NMR signal generated from the subject 130 is detected by the receive coil 110, amplified by a preamplifier (not shown) in the receive coil 110, and transmitted to the receiver 112. In the receiver 112, the amplified NMR signal is subjected to analog-to-digital (AD) conversion and necessary signal processing, thereby generating data. The generated data is transmitted to the control unit 120. This data is also referred to as a received signal or measurement data.

A reception-side cable (not shown) through which the NMR signal received by the receive coil 110 is output is connected to the receive coil 110. Although not shown in Fig. 2, a reception-side connector is connected to an end portion of the reception-side cable. The reception-side connector is connected to a patient table-side connector provided on the patient table 14. The patient table-side connector is connected to a patient table-side cable disposed inside the patient table 14, and the patient table-side cable is connected to the control unit 120. As a result, the receive coil 110, the control unit 120, and the sequencer 118 are communicably connected to each other.

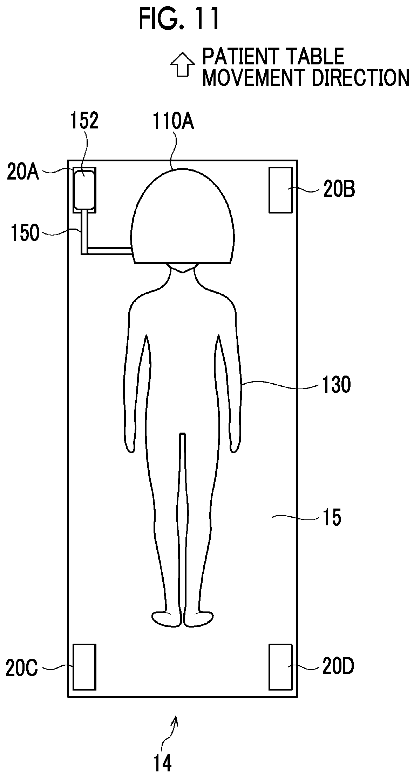

In Fig. 2, although not shown, as examples of the reception-side cable, the reception-side connector, and the patient table-side connector, a reception-side cable 150, a reception-side connector 152, and patient table-side connectors 20A, 20B, 20C, and 20D connected to a receive coil 110A are shown in Fig. 11, which will be described below.

The connection between the receive coil 110, and the control unit 120 and the sequencer 118 is not limited to a wired connection, such as via a cable, and can also be established via a wireless connection. In this case, the receive coil 110 or the patient table 14 further includes at least an AD conversion module and a wireless communication module.

The sequencer 118 controls each unit to operate at a pre-programmed timing and intensity. Among programs, a program that particularly describes the timing and the intensity of the RF pulses, the gradient magnetic field, and signal reception is called a pulse sequence. Various pulse sequences are known depending on the purpose, but detailed descriptions thereof will be omitted here.

The control unit 120 accepts various instruction inputs from the operation unit 122 and integrally controls each unit of the MRI apparatus 10 via the sequencer 118. Prior to the start of MRI imaging, the control unit 120 identifies the body position of the subject 130 based on the camera image captured by the camera 140 and determines consistency by collating the identified body position with the pre-registered body position. The control unit 120 can also identify the body position of the subject 130 based on the coil information such as the type of the receive coil 110 and the position of the patient table-side connector to which the reception-side connector is connected, in addition to the camera image. In the present specification, the body position identified based on the camera image and/or the coil information is referred to as an "identified body position".

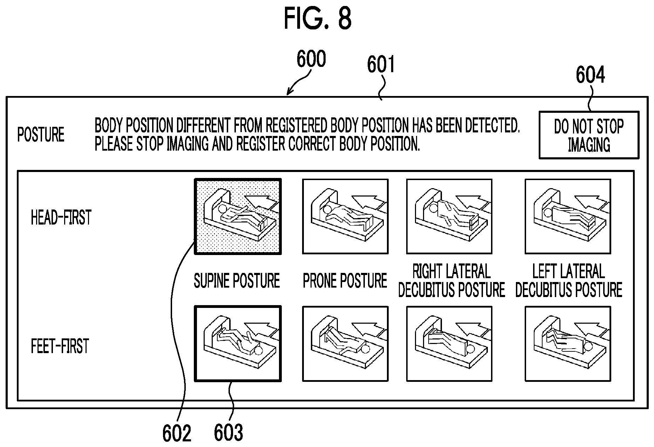

In a case where the registered body position and the identified body position match, MRI imaging is executed, and the control unit 120 performs processing such as inverse Fourier transforming the received signal in a spatial frequency domain received from the receiver 112 and converting the received signal into a real-space image, thereby generating an MRI image. In a case where the registered body position and the identified body position mismatch, the control unit 120 causes a screen for proposing re-registration of the body position based on the identified body position to be displayed as a pop-up. Detailed examples of the pop-up display will be described below with reference to Figs. 8 to 10.

The operation unit 122 includes an input device, such as a mouse and a keyboard, and a display device, such as a liquid crystal display. The operation unit 122 functions as a user interface (UI) for the user such as a technician to launch, stop, and pause the MRI apparatus 10, select a pulse sequence, input imaging conditions or processing conditions, and the like.

The sequencer 118 and the control unit 120 can be configured by using a computer. Additionally, processing functions of the sequencer 118 and the control unit 120 may be implemented by a computer system including a plurality of computers.

### Regarding Camera 140

The camera 140 shown in Fig. 2 is disposed at a position where the camera 140 can image the subject 130 inserted into the imaging space 18. For example, the camera 140 is disposed at a position where the camera 140 captures the subject 130 obliquely from above the subject 130 in the bore, thereby obtaining a video of a relatively wide range including the chest of the subject 130. It is desirable that the camera 140 is configured to image the entire body of the subject 130. It is desirable that a wide-angle lens or a fisheye lens is applied to the imaging optical system of the camera 140.

The camera 140 may be configured to optically capture consecutive images at a frame rate equal to or greater than a predetermined frame rate. For example, it is desirable that the camera 140 can capture 30 or more images per second. The camera 140 may be configured to capture a still image.

The imaging space 18 of the gantry 12 has a structure that makes it difficult for light such as indoor illumination light of the shielded room to enter. Therefore, in a case where the camera 140 that images the inside of the bore is used, it is desirable to dispose an illumination device that irradiates the imaging space 18 with light in order to improve a signal-to-noise ratio (SNR) of the image of the camera 140.

In addition, the camera 140 has a structure that cuts electromagnetic noise such that the gantry 12 does not generate noise due to the electromagnetic noise generated by the camera 140.

Further, the camera 140 has a structure that cuts the static magnetic field generated by the static magnetic field generating magnet 102, the rapidly changing gradient magnetic field applied by the gradient magnetic field coil 104, and the high-frequency magnetic field pulse (RF pulse) emitted from the RF transmit coil 106, so as to function as the camera 140 even in a case where these magnetic fields and high-frequency waves are received.

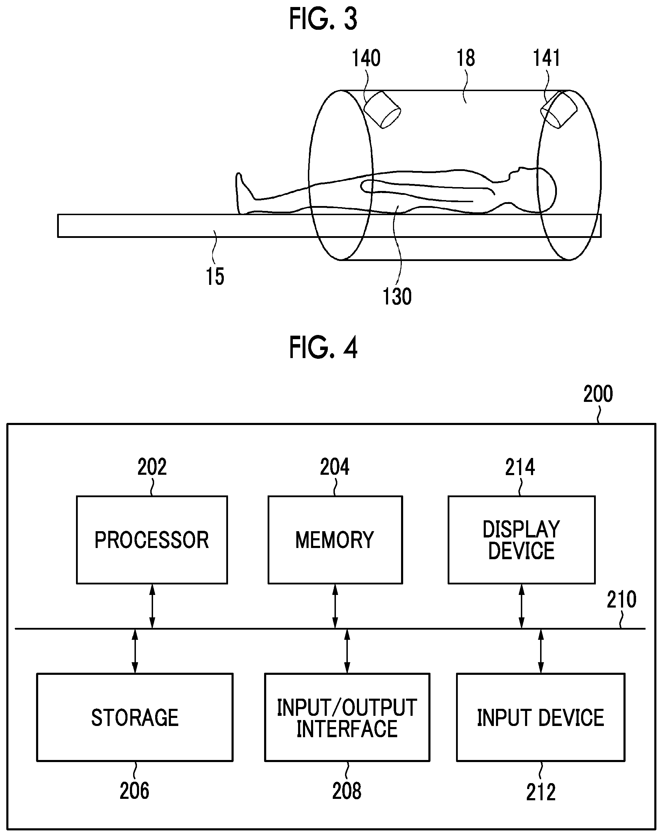

In Fig. 2, an example is shown in which the camera 140 is attached to an entrance side in a case where the subject 130 is inserted into the bore in the gantry 12, but the disposition location and the number of the cameras 140 are not limited to the example of Fig. 2. For example, as shown in Fig. 3, a configuration may be employed in which cameras 140 and 141 are disposed on the entrance side and a far side of the bore, respectively, or a configuration may be employed in which only the camera 141 is disposed on the far side of the bore. By imaging the subject 130 from a plurality of directions using a plurality of cameras, it is possible to accurately identify the body position of the subject 130.

As another example of the camera disposition, instead of or in combination with the cameras 140 and 141 that image the inside of the bore, a camera may be disposed at a position where the camera can image the outside of the bore. For example, a configuration may be employed in which a camera is disposed on an upper part of a front surface (a surface on a patient table side) of the gantry 12, and the camera images the subject 130 before the subject 130 is moved into the bore or while the subject 130 is being moved into the bore. Additionally, the camera may be attached to a ceiling, a wall, or the like of the shielded room.

### Example of Hardware Configuration of Information Processing Apparatus

Fig. 4 is a block diagram showing an example of a hardware configuration of an information processing apparatus 200 used in the MRI apparatus 10. The information processing apparatus 200 may be a personal computer, a workstation, or a server computer. The computer may be a virtual machine. The information processing apparatus 200 is applied to a console of the MRI apparatus 10 and can function as the control unit 120 and the operation unit 122 (refer to Fig. 2). The information processing apparatus 200 may function as the sequencer 118.

The information processing apparatus 200 comprises a processor 202, a memory 204 that is a main storage device, a storage 206 that is an auxiliary storage device, an input/output interface 208, and a bus 210.

The processor 202 includes a central processing unit (CPU). The processor 202 may include a graphics processing unit (GPU). In addition, the processor 202 may include one or a plurality of pieces of hardware of a digital signal processor (DSP), an application-specific integrated circuit (ASIC), a programmable logic device (PLD), and the like.

The processor 202 is connected to the memory 204, the storage 206, the input/output interface 208, an input device 212, and a display device 214, via the bus 210.

The memory 204 includes a random access memory (RAM). The memory 204 may include a read-only memory (ROM). The storage 206 may be, for example, a hard disk drive (HDD), a solid-state drive (SSD), or a combination of a plurality of these. In addition, the storage 206 may include an external storage device such as a removable medium.

The storage device including the memory 204 and the storage 206 stores programs, data, and the like for implementing various functions of the MRI apparatus 10. The processor 202 executes the programs stored in the memory 204 to implement various functions. The processor 202 integrally controls each unit of the information processing apparatus 200, various devices, units, and the like provided in the MRI apparatus 10, and performs various kinds of processing.

The input/output interface 208 includes a communication interface that can be connected to telecommunication lines such as a local area network, a connection interface that can be connected to external devices, and the like. As the connection interface that can be connected to the external devices, for example, a Universal Serial Bus, a High-Definition Multimedia Interface (HDMI) (HDMI is a registered trademark), and the like can be applied.

The processor 202 communicates with various devices of the MRI apparatus 10 via the input/output interface 208, thereby transmitting and receiving necessary information.

The input device 212 includes, for example, a keyboard, a pointing device such as a mouse, a ten-keypad, various switch buttons, and the like. The input device 212 may include a voice input device. Additionally, the input device 212 may be a touch panel-type input device that is configured integrally with a display screen of the display device 214.

The display device 214 is configured, for example, by using a liquid crystal display, an organic electroluminescence (OEL) display, a projector, or an appropriate combination thereof. In addition to the MRI image captured by the MRI apparatus 10, various types of information such as the camera image and the coil information are displayed on the display device 214. The display device 214 is used as a part of the user interface in a case where an input from the input device 212 is accepted. The display device 214 is not limited to a single display device and can also be in a multi-display form comprising a plurality of display devices.

### Overview of Procedure of MRI Imaging

A typical imaging procedure by the MRI apparatus 10 is generally as follows.

[Procedure 1] The user such as a technician uses the input device 212 such as a mouse and a keyboard to register information necessary for the examination, such as the subject information, the imaging site, the body position of the subject 130, and the imaging protocol, on a subject registration screen. The term "registration" includes the concept of "setting". The term "user" includes the concepts of terms such as a technician, an operator, and a manipulator.

The subject information includes the name of the subject, a subject code, and the like. The body position of the subject 130 includes an insertion direction of the subject 130 into the gantry 12 and a posture of the subject 130. The imaging protocol includes an imaging type, an order, and the like. A part or all of the information necessary for these examinations may be manually input by the operator via the input device 212 or may be read from information stored in advance in a recording medium or the like. The operator finally confirms automatically set or input parameters and manually sets the parameters by using the input device 212 as necessary.

[Procedure 2] The subject 130 is placed on the top plate 15 of the patient table 14, and the receive coil 110 is worn by the subject 130. The user connects the reception-side connector of the reception-side cable connected to the receive coil 110 to the patient table-side connector on the top plate 15. The top plate 15 is provided with a plurality of patient table-side connectors, and the reception-side connector is connected to the closest patient table-side connector according to the type of the receive coil 110 to be used and the body position of the subject 130. Depending on the combination of the type of the receive coil 110 and the body position of the subject 130, the position of the patient table-side connector to which the reception-side connector can be connected is restricted.

[Procedure 3] After the receive coil 110 is connected to the patient table-side connector, the top plate 15 is moved to the imaging space 18 by using a gantry operation panel, a foot switch, or the like (not shown), and an examination target site of the subject 130 is moved to a static magnetic field center position of the gantry 12.

[Procedure 4] After that, the user presses a start button for issuing an instruction to start imaging, scanogram imaging for acquiring a positioning image is executed. In the scanogram imaging, a plurality of slices are imaged for one or more cross-sections of an axial cross-section, a coronal cross-section, and a sagittal cross-section. A slice thickness in the scanogram imaging may be set to a value greater than a slice thickness in the main imaging. In a case where the imaging start instruction is accepted, an examination window screen is displayed on the display device 214 of the operation unit 122.

[Procedure 5] After the end of the scanogram imaging, the image for positioning is reconstructed, and the image is displayed on the display device 214.

[Procedure 6] The user sets an imaging position of the main imaging based on a scanogram image. In the MRI apparatus 10, an imaging position including a slice position (cross-section position) to be measured in the main imaging is automatically calculated from the scanogram image, and a recommended imaging position is presented to the user. The user confirms the presented slice position and the like and manually adjusts the imaging position by using the input device 212 of the operation unit 122 as necessary. In addition, the user sets imaging parameters to be applied to the main imaging. A part or all of the imaging parameters may be input by the user via the operation unit 122 or may be automatically set or input. The operator finally confirms the automatically set (automatically input) parameters and manually sets the parameters by using the input device 212 of the operation unit 122 as necessary.

[Procedure 7] The main imaging is executed in accordance with the imaging parameters and the imaging position set in this manner.

[Procedure 8] After the main imaging, the image is reconstructed, and the image is displayed on the display device 214.

[Procedure 9] The user sets a window value to a value suitable for diagnosis for the displayed reconstructed image by using the input device 212 to obtain an image to be used for diagnosis.

[Procedure 10] After the imaging is completed, the top plate 15 is retracted from the imaging space 18, and the subject 130 is removed from the gantry 12. Then, the subject 130 is taken off the patient table 14, and the MRI examination ends.

### Display Example 1 on Console: Example of Subject Registration Screen

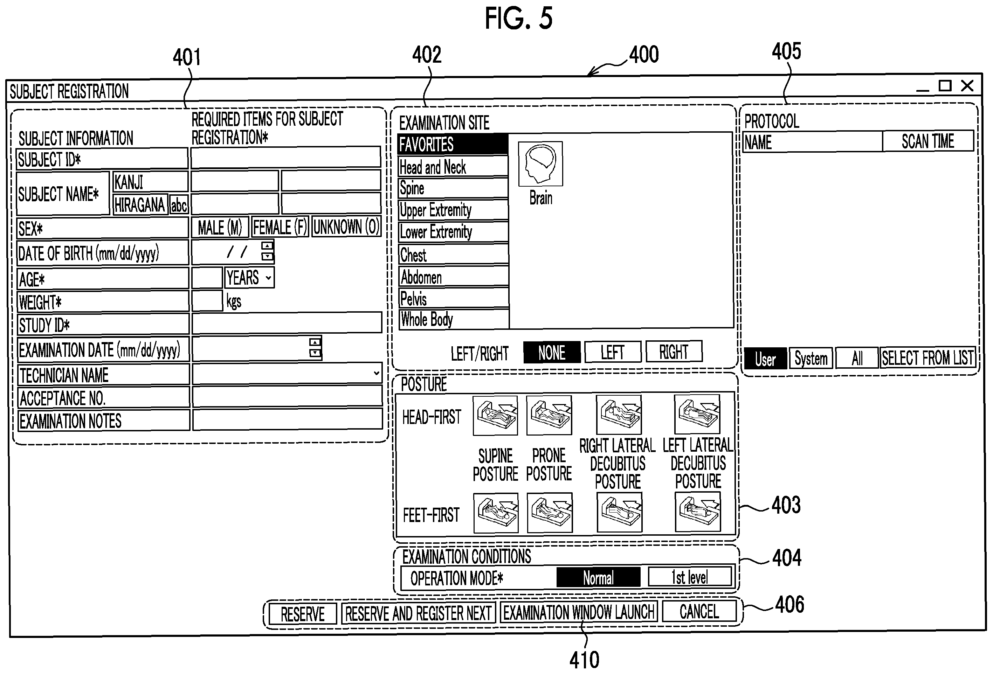

Fig. 5 is an example of a subject registration screen 400. The subject registration screen 400 is a screen for accepting registration of information regarding the subject 130 and examination contents. The subject registration screen 400 includes a subject information area 401 for registering the subject information, an examination site area 402 for registering the examination site, a body position registration area 403 for registering the body position of the subject 130, an examination condition area 404 for registering examination conditions, a protocol area 405 for registering the imaging protocol, and a button section 406.

The subject information area 401 is a region in which information for specifying the subject 130 and information regarding an attribute of the subject 130 are displayed. The examination site area 402 is a region in which information regarding the examination site designated in an examination order is displayed. The body position of the subject 130 is broadly classified into head-first or feet-first, and each of the head-first or feet-first is further classified into a supine posture, a prone posture, a right lateral decubitus posture, or a left lateral decubitus posture.

The body position registration area 403 includes eight icons respectively corresponding to the eight types of body positions, and the body position of the subject 130 is registered by selecting the corresponding icon. The icon of the selected body position is displayed in a display aspect in which the icon is visually distinguished from the other non-selected icons, by means of differentiated display such as highlight display. The body position of the subject 130 may be automatically set in association with the examination site, the imaging protocol, or the like designated in the examination order, or the user may perform an operation of selecting a desired icon.

The examination condition area 404 includes a button for selecting whether the examination is performed in a standard operation mode or in a first-level management operation mode.

In the protocol area 405, the imaging protocol corresponding to an examination instruction designated in the examination order is displayed.

The button section 406 includes, for example, a reservation button, a button for making a reservation and registering the next subject, an examination window launch button 410, a cancel button, and the like. The subject registration screen 400 is an example of a "registration screen" in the present disclosure.

After necessary items are registered on the subject registration screen 400, the screen transitions to the examination window screen in a case where the user presses the examination window launch button 410. It should be noted that the expressions such as "press" or "pressing" for graphical user interface (GUI) buttons include the concepts of operations of inputting a command corresponding to the button, through operations such as clicking, touching, selecting, or designating.

### Display Example 2 on Console: Example of Examination Window Screen

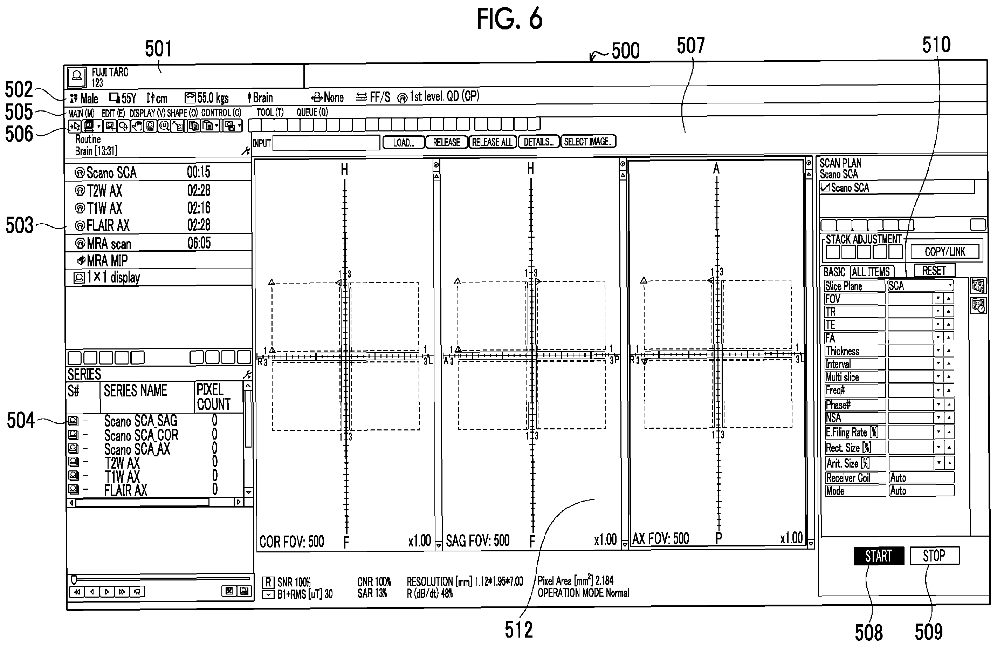

Fig. 6 is an example of an examination window screen 500. The examination window screen 500 is a screen for executing a series of tasks defined in the imaging protocol. The examination window screen 500 includes a switch tab 501, a subject information area 502, a protocol area 503, a series list 504, a menu bar 505, a toolbar 506, a task-specific execution screen 507, a start button 508, a stop button 509, and an imaging condition area 510.

The name of the subject is displayed in a label section of the switch tab 501. In the subject information area 502, attribute information of the subject 130 registered in the subject information area 401 of the subject registration screen 400 is displayed.

The task-specific execution screen 507 includes an image display area 512 in which the MRI image obtained by imaging is displayed. Since the MRI image to be displayed in the image display area 512 has not been obtained before the start of imaging, the image display area 512 may be in a blank state with no MRI image displayed. The MRI image is displayed in the image display area 512 by reading the MRI image obtained by imaging the subject 130. A plurality of images, such as images of different cross-sections, can be displayed side by side in the image display area 512. The image display area 512 shown in Fig. 6 is an example of an area in which each image of three cross-sections: an axial cross-sectional image, a coronal cross-sectional image, and sagittal cross-sectional image, which are positioning images acquired by the scanogram imaging, is read and displayed. The MRI image obtained by imaging may be automatically displayed in the image display area 512.

### Transition Method from Subject Registration Screen 400 to Examination Window Screen 500

The present disclosure is not limited to the form in which the imaging start instruction is input by pressing the examination window launch button 410 in the subject registration screen 400 to transition to the examination window screen 500 and pressing the start button 508 in the examination window screen 500.

The start button 508 may be disposed on the subject registration screen 400, and in a case where the start button 508 is pressed on the subject registration screen 400, the screen may transition to the examination window screen 500.

### Overview of Medical Imaging Support Method According to Embodiment

In a case where the imaging start instruction is issued such as by pressing the start button 508, and the screen has transitioned to the examination window screen 500, the information processing apparatus 200 identifies the actual body position of the subject 130 by using the camera image or the like, stops the imaging in a case where a body position different from the body position of the subject 130 registered in advance is identified, and displays a pop-up for proposing re-registration of the body position on the examination window screen 500.

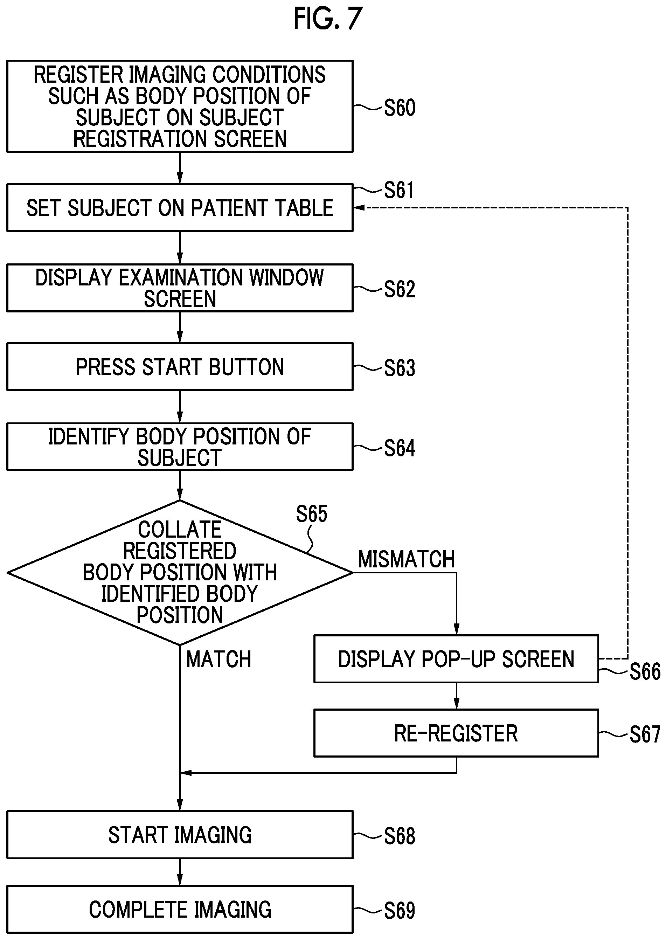

Fig. 7 is a flowchart showing an example of an operation by the MRI apparatus 10. In step S60, the user registers the imaging conditions such as the body position of the subject 130 on the subject registration screen 400. That is, in step S60, the processor 202 displays the subject registration screen 400 on the display device 214 and accepts the registration of the imaging conditions including the body position of the subject.

After registering the information necessary for the examination on the subject registration screen 400, the user sets the subject 130 on the patient table 14 in step S61. That is, as mentioned above, the examination target site of the subject 130 is set to be disposed at the static magnetic field center position of the gantry 12.

After that, in step S62, the processor 202 displays the examination window screen 500 on the display device 214 and accepts the imaging start instruction. The transition from the subject registration screen 400 to the examination window screen 500 may be performed in response to an operation of the user, such as by pressing the examination window launch button 410, or may be automatically performed based on a signal from a sensor or the like that detects opening and closing of a door of the shielded room.

The imaging start instruction may be issued by pressing the start button 508 or may be triggered by operations such as pressing a physical imaging start button on the console, pressing an imaging start button on a gantry monitor, pressing an imaging start button on a gantry operation panel, or closing the door of the shielded room.

In step S63, in a case where the user presses the start button 508 or the like and the processor 202 receives the imaging start instruction, the process proceeds to step S64. The order of steps S60 to S64 can be changed. For example, the examination window screen 500 of step S62 may be displayed immediately after the examination window launch button 410 is pressed at the time of registration in step S60, and then step S61 may be performed. Alternatively, for example, the registration may be performed in step S60 after the subject 130 is set on the patient table 14 in step S61. Additionally, for example, in the case of an emergency patient or the like, two technicians may perform step S60 and step S61 simultaneously. Further, for example, it is also possible to perform step S64 immediately after the subject 130 is set on the patient table 14 in step S61. Furthermore, for example, a start button may be provided on the subject registration screen 400, the imaging start instruction may be accepted on the subject registration screen 400, and the screen may transition to the examination window screen 500 in a case where the processor 202 receives the imaging start instruction.

In step S64, the processor 202 identifies the body position of the subject 130 from the camera image. For example, the processor 202 analyzes the camera image and identifies whether the insertion direction of the subject 130 with respect to the gantry 12 is head-first or feet-first, and whether the posture of the subject 130 is in a supine posture, a prone posture, a left lateral decubitus posture, or a right lateral decubitus posture.

As for the image recognition technology for identifying the body position of the subject 130 from the camera image, for example, a class classification algorithm, an object detection algorithm, a region detection algorithm, or the like using machine learning may be applied, or an image recognition process using pattern matching, a body feature detection process using optical flow calculation or the like, or an appropriate combination of these methods may be applied. As a technology for selecting a region of interest by calculating an optical flow from the camera image, for example, a technology described in JP2022-104170A can be applied. By detecting at least one, preferably a plurality, of body part regions such as the head, the chest, the arms, and the legs of the subject 130 from the camera image, the body position of the subject 130 can be identified based on the distribution of the body part regions indicated by detection results.

In a body position identification process, not only the camera image but also the coil information of the receive coil 110 connected to the MRI apparatus 10 may be used. The coil information includes information on the type of the receive coil 110 and the position of the patient table-side connector to which the reception-side connector is connected, and the like.

The processor 202 can identify the body position of the subject 130 based on at least one of the camera image or connector information. The processor 202 may identify the body position of the subject 130 by using both the camera image and the connector information.

In step S65, the processor 202 collates the registered body position and the identified body position and determines whether or not the registered body position and the identified body position match. In a case where the registered body position and the identified body position match, the process proceeds to step S68, and imaging is started. As a result, imaging is executed in accordance with the imaging protocol.

On the other hand, in a case where it is determined in the determination in step S65 that the registered body position mismatches the identified body position, the process proceeds to step S66.

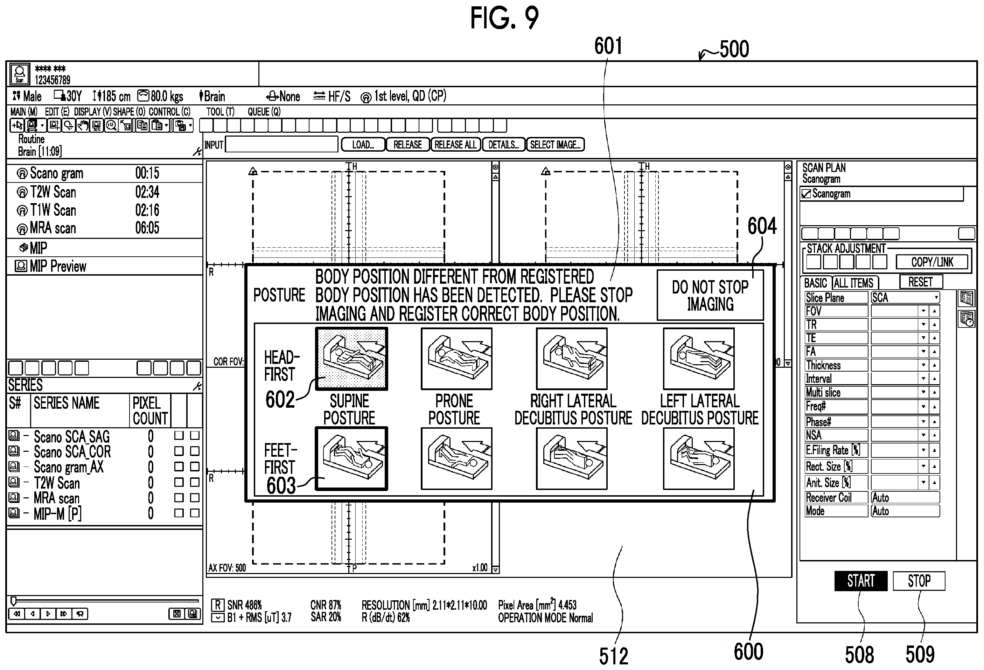

In step S66, the processor 202 displays the pop-up screen for proposing the re-registration of the body position (refer to Figs. 8 and 9). The processor 202 displays, for example, a pop-up screen 600 as shown in Fig. 8 on the examination window screen 500 and prompts the user to re-register the body position on the pop-up screen 600. The term "screen" includes the concept of a video projected onto a display surface of a display device such as the display device 214. In addition, the term "screen" includes not only a full screen that matches the entire physical region of the display surface of the display device, but also the concept of a sub-screen or a sub-window displayed in a region smaller than the full screen.

In step S67, the processor 202 accepts the re-registration of the body position of the subject 130 from the pop-up screen 600. In step S67, in a case where the user confirms the body position of the subject 130 and executes the re-registration of the body position, or in a case where the user issues an instruction to continue the imaging without changing the body position, the process proceeds to step S68, and imaging is started.

In a case where the registered body position is correct, and the body position of the subject 130 actually set on the patient table 14 is incorrect, the user stops the imaging, for example, by pressing the stop button 509. In this case, the process returns to step S61, and the setting of the subject 130 in the imaging space 18 is redone.

After the imaging is started in step S68, the imaging is executed in accordance with the imaging protocol, and in a case where the imaging is completed (step S69), the flowchart of Fig. 7 ends.

### Display Example 1 of Pop-up Screen

Fig. 8 is an example of the pop-up screen 600 that is popped up on the examination window screen 500. The pop-up screen 600 includes an action message 601, a currently registered body position 602, and a proposed body position 603, and prompts the user to confirm and re-register the body position of the subject 130.

The action message 601 is information for notifying the user that the registered body position and the identified body position mismatch and prompting the user to re-register the body position, and may be, for example, a message such as "A body position different from the registered body position has been detected. Please stop imaging and register the correct body position".

The pop-up screen 600 includes a partial screen of the subject registration screen 400 and provides the same GUI as the body position registration area 403 which is a part of the subject registration screen 400. That is, in the pop-up screen 600, icons respectively corresponding to the eight types of body position candidates are displayed as in the body position registration area 403, and the icon of the currently registered body position 602 is displayed in a highlighted manner. Additionally, in the pop-up screen 600, the icon of the proposed body position 603, which is recommended from the identified body position, is displayed in a differentiated manner so as to be visually distinguishable from the currently registered body position 602 and icons other than the proposed body position icon.

Examples of the method for differentiated display may include aspects such as displaying a frame line surrounding the icon in an emphasized manner, making the icon blink, or changing the background color or the like to something different from the other icons to display the icon in a highlighted manner. In this manner, in the pop-up screen 600, it is preferable that all the candidates for the body position are presented, the currently registered body position 602 is displayed using a first differentiation aspect, and the proposed body position 603 is displayed using a second differentiation aspect different from the first differentiation aspect. The proposed body position 603 is proposed as a body position that is recommended for re-registration in place of the currently registered body position 602, and is an example of a "proposal for re-registration" in the present disclosure.

The user can perform the re-registration of a body position consistent with the identified body position by selecting the icon of the proposed body position 603. In a case where the icon of the proposed body position 603 is pressed, a pop-up screen (not shown) for confirmation may be displayed, and an instruction to execute or cancel the re-registration may be accepted.

In addition, the pop-up screen 600 includes an imaging continuation button 604 for continuing imaging without changing the currently registered body position and without stopping the imaging. The imaging continuation button 604 is an example of a "button for issuing an instruction to continue imaging" in the present disclosure.

Although not shown in Fig. 8, either the camera image that is the basis for the proposal or the coil information of the receive coil 110 connected to the patient table 14, or both of these may be displayed on the pop-up screen 600.

### Example of Position of Pop-Up Display on Examination Window Screen

Fig. 9 is a screen example showing an example of a display position of the pop-up screen 600 on the examination window screen 500. The pop-up screen 600 is displayed as a pop-up within the image display area 512 on the examination window screen 500. The image display area 512 is a region disposed in a central part of the examination window screen 500 and is in a blank (no image) state before imaging, with no MRI image displayed.

By displaying the pop-up screen 600 within the image display area 512 in which the MRI image is scheduled to be displayed after imaging, the proposal for re-registration of the body position and the acceptance of re-registration can be performed without hiding setting information such as the subject information, the protocol information, the series information, and the imaging parameters.

The image display area 512 is considered to be a portion of the screen that the user is focused on, where the MRI image acquired by imaging is expected to be displayed. The image display area 512, which is the portion of the screen in the examination window screen 500 that the user is focused on, is a preferred region as a position where the pop-up screen 600 is displayed, because the image display area 512 is the region where the user's attention is directed.

The pop-up screen 600 is not limited to being displayed as a single pop-up window and may be displayed in a display form in which the pop-up screen 600 is separated into a plurality of windows or GUI components. For example, the action message 601 may be displayed in a separate window from the re-registration window of the body position, and the GUI components such as the imaging continuation button 604 may be disposed outside the re-registration window of the body position.

### Display Example 2 of Pop-up Screen

In Figs. 8 and 9, an example has been described in which the pop-up screen 600 as a sub-window smaller than the examination window screen 500 is displayed in a superimposed manner on a part of the examination window screen 500, but instead of such a pop-up screen 600, the pop-up may be displayed in full screen. For example, the pop-up screen 600 as shown in Fig. 9 may be displayed in full screen, or a different pop-up screen including a part or all of display contents of the pop-up screen 600 may be displayed in full screen.

As the pop-up screen, the subject registration screen 400 may be displayed in full screen. In this case, in the body position registration area 403 of the subject registration screen 400 that is popped up, as in the example of Fig. 8, the action message 601 is displayed, and the icon of the proposed body position 603 and the currently registered body position 602 are displayed in a contrastingly differentiated manner.

### Regarding Display Indicating That Body Position Is Being Identified Based on Camera Image

In a state after the transition to the examination window screen 500, during a period until the identification of the subject's body position based on the camera image is completed, for example, information indicating that identification is being performed based on the camera image, such as "detecting patient orientation", may be displayed on the examination window screen 500.

### Example of Case Where Body Position Cannot Be Identified from Camera Image

In a case where the body position of the subject 130 cannot be identified from the camera image, instead of displaying the pop-up screen 600 including the proposed body position 603, the camera image may be displayed on the examination window screen 500 as a pop-up. In this case, it is preferable to display not only the camera image but also the coil information in a case where there is coil information, in addition to the camera image.

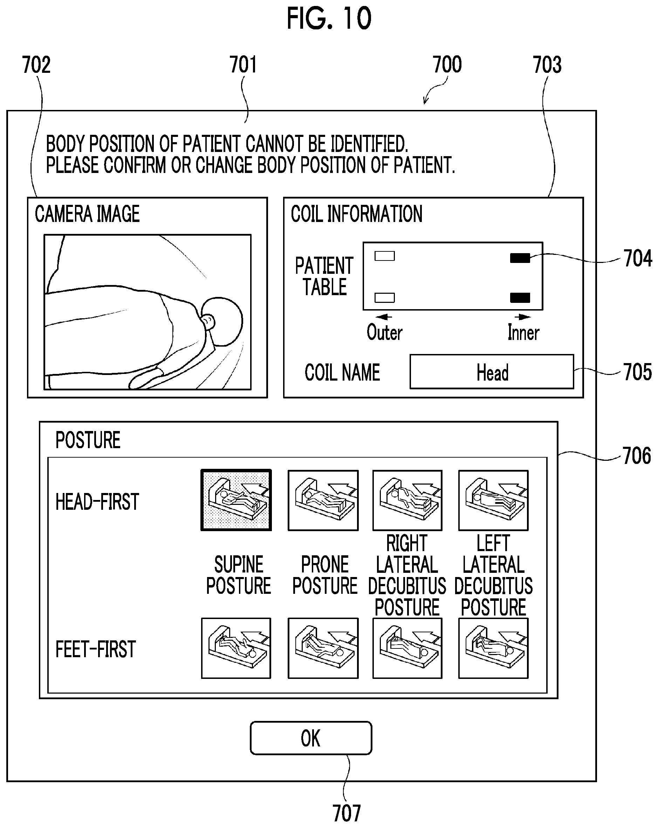

Fig. 10 is a display example of a screen including the camera image and the coil information. In a case where the body position of the subject 130 cannot be identified based on the camera image, the processor 202 displays, for example, a pop-up screen 700 as shown in Fig. 10 on the examination window screen 500 as a pop-up. The pop-up screen 700 is an example of a "screen including the information obtained from the sensor" in the present disclosure.

The pop-up screen 700 includes an action message 701, a camera image 702, coil information 703, a body position registration area 706, and an OK button 707.

The action message 701 is information for notifying the user that the body position cannot be identified and prompting the user to confirm the body position, and may display, for example, a message such as "The body position of the patient cannot be identified. Please confirm or change the body position of the patient".

A live video captured by the camera 140 may be displayed in a display area of the camera image 702. For example, a position of a patient table-side connector 704 to which the reception-side connector of the receive coil 110 is connected may be shown in a display area of the coil information 703. Additionally, a coil name 705 indicating the type of the receive coil 110 being used may be displayed in the display area of the coil information 703.

As the coil information 703, both the information indicating the position of the patient table-side connector 704 to which the reception-side connector is connected and the coil name 705 may be displayed, or only one of them may be displayed.

The body position registration area 706 is a screen that provides the same GUI as the body position registration area 403 of the subject registration screen 400, and the icon of the currently registered body position is displayed in a highlighted manner. Icons of other body positions are displayed as candidates for re-registration. The user can select an icon of a desired body position from the body position registration area 706 to change the registered body position.

The OK button 707 is a button for closing the pop-up screen 700. The user may press the OK button 707 after changing the registered body position or may press the OK button 707 without changing the registered body position. In a case where the OK button 707 is pressed, imaging is started.

### Example of Method of Identifying Body Position Based on Coil Information

### In Case Where Neuro Coil for Head and Neck Is Used

Fig. 11 is a schematic plan view of the patient table 14 on which the subject 130 wearing the receive coil 110A, which is a Neuro Coil for the head and neck, is placed. An upward direction of Fig. 11 is a patient table movement direction in a case where the subject 130 is advanced into the bore of the gantry 12. The top plate 15 is provided with a plurality of patient table-side connectors 20A, 20B, 20C, and 20D. Although Fig. 11 shows an example in which the patient table-side connectors 20A, 20B, 20C, and 20D are disposed at four corners of the top plate 15, the number and disposition of the patient table-side connectors are not limited to the form shown in Fig. 11. The patient table-side connectors 20A, 20B, 20C, and 20D are examples of a "plurality of connectors" in the present disclosure.

The patient table-side connectors 20A, 20B, 20C, and 20D have the same shape, and the reception-side connector 152 of the receive coil 110 can be connected thereto. Fig. 11 shows a state in which the receive coil 110A, which is a coil for the head and neck, is connected to the patient table-side connector 20A via the reception-side cable 150 and the reception-side connector 152.

The receive coil 110A for the head and neck is specifically designed for the supine posture and is specified to be fixed at a position on the patient table 14 that is close to the gantry 12. The user can connect the reception-side connector 152 of the receive coil 110A to the patient table-side connector 20A or the patient table-side connector 20B on the top plate 15.

In a case where the reception-side connector 152 of the receive coil 110A is connected to any of the patient table-side connector 20A, 20B, 20C, or 20D, the processor 202 acquires information indicating the type of the connected receive coil 110A and the position of the connected patient table-side connector and determines the body position of the subject 130 based on the acquired coil information.

The receive coil 110A connected to the patient table 14 may be automatically recognized by a function conforming to plug-and-play, and the position of the connected patient table-side connector 20A, 20B, 20C, or 20D may be specified. In addition, for example, each of the patient table-side connectors 20A, 20B, 20C, and 20D may be connected to a connection detection circuit that detects connection of the reception-side connector 152, and the processor 202 may detect the position of the patient table-side connector 20A, 20B, 20C, or 20D to which the reception-side connector 152 is connected, based on a signal from the connection detection circuit.

The processor 202 may acquire the information indicating the type of the receive coil 110A connected to the patient table 14 through digital communication or may acquire the information through barcode reading, electronic tag reading, image recognition, or the like. A circuit that generates a signal required for automatically recognizing the receive coil 110A connected to the patient table 14, a connection detection circuit, an information reading device, and the like are examples of a "sensor that acquires information used to identify the body position of the subject" in the present disclosure.

The term "sensor" is interpreted broadly, and a mechanism that automatically recognizes the connected receive coil 110A or that detects the patient table-side connector 20A, to which the receive coil 110A is connected, by exchanging information between devices upon connector connection is included in the concept of the "sensor".

A correspondence relationship between the coil information and the body position of the subject 130 can be defined in advance by using a table or the like based on the specification of the receive coil 110. A table that defines such a correspondence relationship is stored in the memory 204 or the like, and the processor 202 can identify the body position of the subject 130 by referring to the correspondence relationship table from the acquired coil information.

From the specification of the Neuro Coil for the head and neck, as shown in Fig. 11, in a case where the receive coil 110A is connected to the patient table-side connector 20A, the body position of the subject 130 is identified as head-first and in a supine posture. The receive coil 110A can also be connected to the patient table-side connector 20B, and similarly, in a case where the receive coil 110A is connected to the patient table-side connector 20B, the body position is identified as head-first and in a supine posture.

In Fig. 11, the Neuro Coil is illustrated, but the type of the receive coil 110 may include a spine coil, a torso coil, a foot ankle coil, a hand coil, a shoulder coil, a breast coil, and the like, in addition to the Neuro Coil. Depending on the type of the receive coil 110, it may be difficult to uniquely identify the body position. Therefore, it is preferable to identify the body position of the subject 130 by using the camera image and the coil information in combination.

### Functional Configuration of Information Processing Apparatus 200

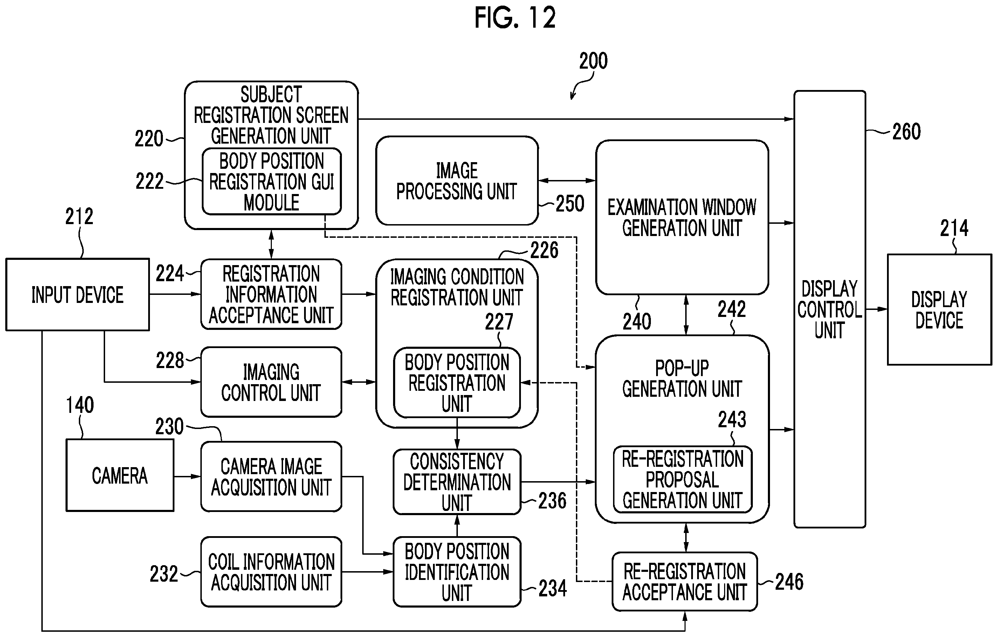

Fig. 12 is a block diagram showing a functional configuration of the information processing apparatus 200 that performs a medical imaging support method according to the embodiment.

The information processing apparatus 200 comprises a subject registration screen generation unit 220, a registration information acceptance unit 224, an imaging condition registration unit 226, an imaging control unit 228, a camera image acquisition unit 230, a coil information acquisition unit 232, a body position identification unit 234, a consistency determination unit 236, an examination window generation unit 240, a pop-up generation unit 242, a re-registration acceptance unit 246, an image processing unit 250, and a display control unit 260.

The functions of each processing unit in the information processing apparatus 200 may be implemented by the processor 202 executing the program stored in the memory 204.

The subject registration screen generation unit 220 generates the subject registration screen 400. The subject registration screen generation unit 220 includes a body position registration GUI module 222. The body position registration GUI module 222 is a module that configures a GUI of a body position registration area on the subject registration screen 400, and includes components that implement the display of icons respectively corresponding to the eight types of body positions.

The registration information acceptance unit 224 accepts information to be pre-registered through the subject registration screen 400. The user can operate the input device 212 to input necessary information. In addition, the registration information acceptance unit 224 may acquire a part of the imaging conditions from an electronic medical record system, an ordering system, a radiology information system (RIS), or the like (not shown).

The imaging condition registration unit 226 includes a body position registration unit 227 and registers the imaging conditions including the examination site, the imaging protocol, the subject's body position, and various imaging parameters acquired via the registration information acceptance unit 224. The pre-registered body position (registered body position) is registered in the body position registration unit 227.

The examination window generation unit 240 generates the examination window screen 500. The camera image acquisition unit 230 acquires the camera image from the camera 140. The coil information acquisition unit 232 acquires the coil information of the receive coil 110 connected to the patient table 14.

The body position identification unit 234 identifies the body position of the subject 130 based on at least one of the camera image or the coil information. The body position identification unit 234 includes an image recognition processing unit that analyzes the camera image to identify the body position of the subject 130. For the image recognition technology for identifying the body position of the subject 130 from the camera image, for example, a machine learning algorithm can be applied. That is, the body position identification unit 234 may include a trained model that has been trained through machine learning to identify the body position of the subject 130 from the camera image. The trained model may be, for example, a classification model that is configured by using a convolutional neural network and that performs class classification of the body positions. The trained model is not limited to the classification model, and may be, for example, a model that performs an object detection task or a region classification (semantic segmentation) task and may recognize sites such as the head, the abdomen, the arms, and the legs of the subject 130 from the camera image and identify the body position based on a body structure. It should be noted that the trained model is essentially a program.

Additionally, the body position identification unit 234 may calculate an optical flow indicating the movement of the subject 130 between frames based on the camera image and identify the body position of the subject 130 based on a calculation result of the optical flow.

Further, the body position identification unit 234 can also identify the body position of the subject 130 from the coil information. The body position identification unit 234 has a table in which a correspondence relationship between a combination of the type of the receive coil and the position of the patient table-side connector to which the reception-side connector is connected, and the subject's body position is defined, and can identify the body position by referring to the table from the acquired coil information.

The consistency determination unit 236 determines consistency by collating the registered body position registered in the body position registration unit 227 with the identified body position identified by the body position identification unit 234.

The pop-up generation unit 242 generates the pop-up screen 600 and the pop-up screen 700. The pop-up generation unit 242 outputs the pop-up screen 600 on the examination window screen 500 in a case where the consistency determination unit 236 determines that the registered body position and the identified body position mismatch. The pop-up generation unit 242 includes a re-registration proposal generation unit 243. The re-registration proposal generation unit 243 displays the body position corresponding to the identified body position identified by the body position identification unit 234 as a proposed body position on the pop-up screen 600. In addition, the pop-up generation unit 242 outputs the pop-up screen 700 on the examination window screen 500 in a case where the body position of the subject 130 cannot be identified by the body position identification unit 234.

The re-registration acceptance unit 246 accepts the re-registration of the body position of the subject 130 through the pop-up screen 600 or the pop-up screen 700. The user can perform the re-registration from the pop-up screen 600 by operating the input device 212.

The imaging control unit 228 controls MRI imaging in accordance with an instruction from the input device 212. The imaging control unit 228 executes MRI imaging based on the imaging conditions registered in the imaging condition registration unit 226.

The image processing unit 250 performs an image reconstruction process from the measurement data obtained by imaging the subject 130 to generate the MRI image. The MRI image generated by the image processing unit 250 is read by the examination window generation unit 240 and output to the image display area 512.

The display control unit 260 generates data for display on the display device 214. The data for display, such as the subject registration screen 400 generated by the subject registration screen generation unit 220, the examination window screen 500 generated by the examination window generation unit 240, and the pop-up screen 600 or the pop-up screen 700 generated by the pop-up generation unit 242, is displayed on the display device 214 via the display control unit 260.

### Regarding Processor

In the present embodiment, each processing is executed by any computer. Additionally, any computer may execute the processing through a processor, a program, or a combination thereof. Any computer may be a general-purpose computer, a computer for a specific application, a system such as a workstation, or other hardware elements capable of executing a program.

The processor may be configured by using one or a plurality of pieces of hardware, and the type of hardware is not limited. For example, the processor may be configured by using hardware such as a central processing unit (CPU), a micro processing unit (MPU), a programmable logic device such as a field programmable gate array (FPGA), a dedicated circuit for executing specific processing such as an application-specific integrated circuit (ASIC), a graphic processing unit (GPU), or a neural processing unit (NPU).

In addition, the processor includes units or means that execute various types of processing in the present embodiment. Further, the type of hardware may be a combination of different types of hardware. In a case where a plurality of pieces of hardware are configured to execute one or a plurality of pieces of processing of a certain processor, the plurality of pieces of hardware may be present in devices physically separated from each other or may be present in the same device. Additionally, in any of the embodiments, the order of each processing by the processor is not limited to the above-described order and may be changed as appropriate. The hardware is configured by using an electrical circuit (circuitry) in which circuit elements such as semiconductor elements are combined.

Further, the present embodiment may be implemented by hardware, software, firmware, microcode, or a combination thereof. Software, firmware, and microcode are configured by programs. In addition, the program may be, for example, a program module group, and each of functions thereof may be implemented by a processor configured to execute each function. The program may be a program code or a plurality of code segments stored in one or a plurality of non-transitory computer-readable media (for example, a storage medium, other storages, or the like). The program may be stored in a plurality of non-transitory computer-readable media present in devices physically separated from each other. The program code or the code segment may represent any combination of procedures, functions, subprograms, routines, subroutines, modules, software packages, classes, or commands, data structures, or program statements. The program code or the code segment may be connected to other code segments or hardware circuits by transmitting and receiving information, data, arguments, parameters, or memory contents.

### Application Example to Program and Program Product

The medical imaging support method according to the embodiment may be configured as a program or a program product for causing a processor or a computer including the processor to implement the functions of each process. The program product is a computer-readable medium that is a tangible and non-transitory information storage medium on which a program is recorded.

A program for causing a computer to implement a part or all of the processing functions of the information processing apparatus 200 can be recorded on a computer-readable medium, which is a non-transitory storage medium such as an optical disk, a magnetic disk, a semiconductor memory, or other tangible media, and the program can be provided through this storage medium.

Additionally, instead of the aspect in which the program is stored in such a tangible and non-transitory computer-readable medium and then provided, program signals can also be provided as a download service using telecommunication lines such as the Internet.

Further, a part or all of the processing functions of the information processing apparatus 200 may be implemented using cloud computing or may be provided as software as a service (SaaS).

### Effects of Embodiment

With the MRI apparatus 10 according to the embodiment, the following effects can be obtained.
(1) In a case where the screen has transitioned from the subject registration screen 400 to the examination window screen 500, and a body position different from the registered body position is identified based on the camera image or the like, the pop-up screen 600 including the proposed body position 603 for re-registration is displayed on the examination window screen 500. As a result, the user can easily perform the re-registration from the pop-up screen 600 by determining the appropriateness of the proposed body position 603.
(2) In the pop-up screen 600, since the proposed body position 603 reflecting the identified body position is presented, the user can re-register the appropriate body position consistent with the identified body position with a simple operation.
(3) The re-registration of the body position can be performed from the pop-up screen 600 on the examination window screen 500 without returning to the subject registration screen 400 from the examination window screen 500, thereby improving convenience.
(4) Since the pop-up screen 600 provides the same GUI as the body position registration area 403 of the subject registration screen 400, the user can re-register the body position without any discomfort, using the same intuitive operation as on the subject registration screen 400.
(5) With the MRI apparatus 10, user convenience is improved, and it is possible to capture the MRI image of the subject 130 in an appropriate body position.

### Modification Example 1

The pop-up screen 600 may include the stop button 509 for issuing an instruction to stop imaging. In addition, the pop-up screen 600 may include the same button as the OK button 707.

### Modification Example 2

The MRI apparatus 10 is an example of a medical imaging apparatus. The technology of the present disclosure can be applied not only to a tunnel-type MRI apparatus 10 having a cylindrical bore but also to, for example, a medical imaging apparatus such as an open-type MRI apparatus having an open bore, a computed tomography (CT) apparatus, a positron emission tomography (PET) apparatus, or an X-ray fluoroscopic diagnostic apparatus. The medical image obtained by the medical imaging apparatus may be a two-dimensional image or a three-dimensional image.

### Modification Example 3

In the above-mentioned embodiment, eight types of body positions are exemplified as the candidates for the body position of the subject 130, but the candidates for the body position may be only a part of these depending on the type of medical imaging apparatus. For example, only two types of body positions, such as head-first and feet-first, may be used.

### Others

The present invention is not limited to the above-mentioned embodiment, and various modifications are possible within the scope of the technical concept of the present disclosure without departing from its gist.

### Explanation of References

10: MRI apparatus
12: gantry
14: patient table
15: top plate
16: leg portion
18: imaging space
20A, 20B, 20C, 20D: patient table-side connector
102: static magnetic field generating magnet
104: gradient magnetic field coil
106: RF transmit coil
110: receive coil
110A: receive coil
112: receiver
114: gradient magnetic field power supply
116: high-frequency magnetic field generator
118: sequencer
120: control unit
122: operation unit
130: subject
140, 141: camera
150: reception-side cable
152: reception-side connector
200: information processing apparatus
202: processor
204: memory
206: storage
208: input/output interface
210: bus
212: input device
214: display device
220: subject registration screen generation unit
222: body position registration GUI module
224: registration information acceptance unit
226: imaging condition registration unit
227: body position registration unit
228: imaging control unit
230: camera image acquisition unit
232: coil information acquisition unit
234: body position identification unit
236: consistency determination unit
240: examination window generation unit
242: pop-up generation unit
243: re-registration proposal generation unit
246: re-registration acceptance unit
250: image processing unit
260: display control unit
400: subject registration screen
401: subject information area
402: examination site area
403: body position registration area
404: examination condition area
405: protocol area
406: button section
410: examination window launch button
500: examination window screen
501: switch tab
502: subject information area
503: protocol area
504: series list
505: menu bar
506: toolbar
507: task-specific execution screen
508: start button
509: stop button
510: imaging condition area
512: image display area
600: pop-up screen
601: action message
602: body position
603: proposed body position
604: imaging continuation button
700: pop-up screen
701: action message
702: camera image
703: coil information
704: patient table-side connector
705: coil name
706: body position registration area
707: OK button
S60 to S69: steps of operation of MRI apparatus

## Claims

1. An information processing apparatus comprising:
a processor,
wherein the processor is configured to:
display a registration screen for accepting registration of a body position of a subject on a display device;
accept an imaging start instruction for capturing a medical image of the subject whose body position has been registered;
identify the body position of the subject based on information obtained from a sensor; and
in a case where the registration screen has transitioned to an examination window screen including an image display area for displaying the medical image of the subject, and the identified body position and the registered body position are different from each other, display a pop-up screen including a proposal for re-registration of the body position on the examination window screen.

2. The medical imaging apparatus according to claim 1,
wherein the pop-up screen includes a part of the registration screen.

3. The medical imaging apparatus according to claim 1 or 2,
wherein the processor is configured to:
accept re-registration of the body position of the subject on the pop-up screen.

4. The medical imaging apparatus according to any one of claims 1 to 3,
wherein the registration screen is a screen for accepting registration of imaging conditions including the body position of the subject and registration of subject information.

5. The medical imaging apparatus according to claim 1 or 2,
wherein the registered body position and a proposed body position for re-registration are displayed on the pop-up screen.

6. The medical imaging apparatus according to any one of claims 1 to 5,
wherein the processor is configured to:
display a proposed body position corresponding to the identified body position on the pop-up screen.

7. The medical imaging apparatus according to any one of claims 1 to 6,
wherein the processor is configured to:
in a case where the examination window screen is being displayed, display information indicating that identification of the body position of the subject is being performed on the examination window screen during a period until the identification of the body position is completed.

8. The medical imaging apparatus according to any one of claims 1 to 7,
wherein the processor is configured to:
in a case where the body position of the subject is not identified, display a screen including the information obtained from the sensor on the examination window screen as a pop-up.

9. The medical imaging apparatus according to claim 8,
wherein the information obtained from the sensor includes an image captured by a camera as the sensor,
and/or,
wherein the information obtained from the sensor includes
coil information including at least one of information indicating a type of a receive coil that receives a signal emitted by the subject or information indicating a position of a connector to which the receive coil is connected.

10. The medical imaging apparatus according to claim 8 or 9,
wherein the screen including the information obtained from the sensor displays
the registered body position, and
a body position as a candidate for re-registration.

11. A medical imaging apparatus that captures a medical image of a subject, comprising:
a sensor that acquires information used to identify a body position of the subject; and
the medical imaging apparatus according to any one of claims 1 to 10.

12. The medical imaging apparatus according to claim 11,
wherein the sensor includes a camera, and
the information used to identify the body position of the subject includes an image captured by the camera.

13. The medical imaging apparatus according to claim 11 or 12, further comprising:
a patient table including a top plate that moves with the subject placed thereon; and
a gantry including an imaging space into which the subject is inserted by movement of the top plate, and
wherein preferably, the patient table includes a plurality of connectors to which a receive coil that receives a signal emitted by the subject is connected, and
the sensor detects at least one of a position of the connector to which the receive coil is connected or a type of the receive coil.

14. A medical imaging support method executed by a processor, comprising:
causing the processor to execute:
displaying a registration screen for accepting registration of a body position of a subject on a display device;
accepting an imaging start instruction for capturing a medical image of the subject whose body position has been registered;
identifying the body position of the subject based on information obtained from a sensor; and
in a case where the registration screen has transitioned to an examination window screen including an image display area for displaying the medical image of the subject, and the identified body position and the registered body position are different from each other, displaying a pop-up screen including a proposal for re-registration of the body position on the examination window screen.

15. A non-transitory, computer-readable recording medium which records thereon a program for causing a computer to execute the medical imaging support method according to claim 14.
